(19) Europäisches Patentamt
European Patent Office
Office européen des brevets

(11) **EP 1 280 484 B2**

(12) # NEUE EUROPÄISCHE PATENTSCHRIFT
Nach dem Einspruchsverfahren

(45) Veröffentlichungstag und Bekanntmachung des Hinweises auf die Entscheidung über den Einspruch:
**27.02.2013 Patentblatt 2013/09**

(45) Hinweis auf die Patenterteilung:
**27.06.2007 Patentblatt 2007/26**

(21) Anmeldenummer: **01936314.2**

(22) Anmeldetag: **03.05.2001**

(51) Int Cl.:
***A61F 9/013*** (2006.01)

(86) Internationale Anmeldenummer:
**PCT/EP2001/004978**

(87) Internationale Veröffentlichungsnummer:
**WO 2001/085075 (15.11.2001 Gazette 2001/46)**

(54) **Vorrichtung zum Führen eines Laserstrahls über die Hornhaut eines Auges und Verfahren zum Erzeugen eines entsprechenden Steuerprogramms**

Device for guiding a laser beam over the cornea of an eye and a method for creating a control program therefore

Dispositif pour guider un rayon laser au-dessus de la cornée d'un oeil et une méthode pour créer un programme de gestion correspondant

(84) Benannte Vertragsstaaten:
**DE ES FR IT**

(30) Priorität: **11.05.2000 DE 10022995**

(43) Veröffentlichungstag der Anmeldung:
**05.02.2003 Patentblatt 2003/06**

(73) Patentinhaber: **WaveLight Laser Technologie AG 91058 Erlangen (DE)**

(72) Erfinder:
• **MROCHEN, Michael**
  **01279 Dresden (DE)**
• **KAEMMERER, Maik**
  **01161 Dresden (DE)**
• **SEILER, Theo**
  **95239 Zell (DE)**

(74) Vertreter: **von Hellfeld, Axel**
**Wuesthoff & Wuesthoff**
**Patent- und Rechtsanwälte**
**Schweigerstrasse 2**
**81541 München (DE)**

(56) Entgegenhaltungen:
EP-A2- 1 040 797     WO-A-01/50945
WO-A2-01/50945       DE-C- 19 702 335
DE-C1- 19 702 335    US-A- 5 219 344
US-A- 5 425 727      US-A- 5 425 727

• 'Höhere Mathematik griffbereit', 1972, AKADEMIE VERLAG Seiten 638 - 643
• MAX BORN: 'Principles of Optics', Bd. 6, 1993, PERGAMON PRESS Seiten 37 - 45
• BERGMANN-SCHÄFER: 'Experimentalphysik', Bd. 3, 1987, DE GRUYTER Seite 496

EP 1 280 484 B2

**Beschreibung**

Die Erfindung betrifft eine Vorrichtung zum Führen eines Laserstrahls orts- und zeitgesteuert über eine zu korrigierende Hornhaut.

Die photorefraktive Keratektomie (englisch: Photorefractive Keratectomy) ist bisher ein weitgehend etabliertes Verfahren zur Korrektur von Fehlsichtigkeit niederer Ordnung, also zum Beispiel von Myopie, Hyperopie, Astigmatismus, myopem Astigmatismus und hyperopem Astigmatismus. Der Begriff "phororefraktive Keratektomie (PRK)" wird üblicherweise dahingehend verstanden, daß damit nur ein Eingriff an der Hornhautoberfläche gemeint ist, nachdem das sog. Hornhautepithel entfernt ist. Nach Entfernung des Epithels liegt die Bowman-Membran bzw. das Hornhautstroma frei und kann mit einem Laser abgetragen werden. Von der PRK im allgemeinen unterschieden wird das LASIK-Verfahren (Laser In Situ Keratomileusis). Beim LASIK-Verfahren wird zunächst mit einem sog. Mikrokeratom ein ca. 100 $\mu$m bis 200 $\mu$m dickes Hornhautscheibchen (sog. "Flap") mit einem Durchmesser von 8 bis 10 mm bis auf einen geringen, als "Scharnier" dienenden Rest abgeschnitten. Dieses Scheibchen (Flap) wird zur Seite geklappt, und danach erfolgt die Ablation (Entfernung) von Material mittels Laserstrahlung direkt im Stroma, also nicht an der Hornhautoberfläche. Nach der Laserbehandlung wird der Deckel wieder an seinen ursprünglichen Platz zurückgeklappt, und es erfolgt in der Regel eine relativ schnelle Heilung.

Die nachfolgend beschriebene Erfindung eignet sich sowohl für die vorstehend erläuterte PRK als auch, insbesondere, für die LASIK-Technik.

Bei der PRK und bei LASIK wird Material der Hornhaut abgetragen. Der Abtrag ist eine Funktion der auf die Hornhaut auftreffenden Energiedichte (Energie pro Flächeneinheit) des Laserstrahls. Es sind unterschiedliche Techniken für die Strahlformung und Strahlführung bekannt, so zum Beispiel die sogenannte Schlitz-Abtastung (slit scanning), bei der die Strahlung mittels eines bewegten Schlitzes über den zu bearbeitenden Bereich geführt wird, das sogenannte Fleck-Abtasten (scanning-spot), bei dem ein Strahlungsfleck mit sehr geringen Abmessungen über das abzutragende Gebiet geführt wird, und auch die sogenannte Vollabtragung (fufl-abladon oder widefield ablation), bei der die Strahlung großflächig über den gesamten abzutragenden Bereich eingestrahlt wird und wobei die Energiedichte sich über das Strahlprofil ändert, um den gewünschten Abtrag der Hornhaut zu erreichen. Der Stand der Technik kennt für die genannten Strahl-Führungen jeweils geeignete Algorithmen zum Steuern der Strahlung, um die Hornhaut so abzutragen, dass die Cornea schließlich den gewünschten Krümmungsradius erhält.

Das vorstehend bereits erwähnte "Fleck-Abtasten" (scanning- spot) verwendet einen auf einen relativ kleinen Durchmesser (0,1-2mm) fokussierten Laserstrahl, der mittels einer Strahlführungseinrichtung auf verschiedene Stellen der Hornhaut gerichtet und durch einen sogenannten Abtaster (scanner) sukzessive so bewegt wird, daß letztlich der gewünschte Abtrag von der Cornea erreicht wird. Die Abtragung erfolgt also gemäß einem sogenannten Ablationsprofil. Bei der PRK und LASIK sind insbesondere sogenannte galvanometrische Abtaster (Scanner) verwendbar (vgl. Aufsatz G.F. Marshall in LASER FOCUS WORLD, Juni 1994, S. 57). Es sind inzwischen auch andere Scan-Techniken bekannt für die Führung des Laserstrahls.

Nach dem Stand der Technik werden zur Zeit die genannten Fehlsichtigkeiten niederer Ordnung (z.B. Myopie, Hyperopie, Astigmatismus) nach den sogenannten Refraktionsdaten des Patientenauges durchgeführt, d.h. der für das Patientenauge gemessene Dioptrie-Wert bestimmt das Ablationsprofil, gemäß dem Material von der Hornhaut abgetragen (ablatiert) wird (vgl. T. Seiler und J. Wollensak in LASERS AND LIGHT IN OPTHALMOLOGY, Vol. 5, Nr. 4, S. 199-203, 1993). Gemäß diesem Stand der Technik wird also für ein gegebenes Patientenauge mit einem bestimmten Dioptrie-Wert die Laserstrahlung so über die Hornhaut (Cornea) geführt, daß ein vorgegebenes Ablationsprofil abgetragen wird, zum Beispiel entsprechend einer Parabel bei einer Myopiekorrektur. Mit anderen Worten: das Ablationsprofil ist nur gemäß dem Dioptrie-Wert an das individuelle Auge angepaßt nicht aber gemäß lokalen Unregelmäßigkeiten des optischen Systems "Auge".

Auch der Aufsatz von J.K. Shimmick, W.B. Telfair et al. in JOURNAL OF REFRATIVE SURGERY, Vol. 13, Mai/Juni 1997, S. 235-245, beschreibt die Korrektur von Sehfehlern niederer Ordnung mittels photorefraktiver Keratektomie, wobei die Photoablationsprofile theoretischen Parabelformen entsprechen. Es wurde darüber hinaus dort nur vorgeschlagen, einige empirische Korrekturfaktoren in das Ablationsprofil einzufügen, die der Wechselwirkung zwischen Laser und Gewebe Rechnung tragen, um im Ergebnis eine paraboloidförmige Abtragung auf dem Auge zu erreichen.

Ein besonderes Problem bei der photorefraktiven Keratektomie und LASIK ist die relative Positionierung von Laserstrahl und Auge. Der Stand der Technik kennt verschiedene Verfahren hierfür, so zum Beispiel sogenannte "Eye-tracker", d.h. Einrichtungen, die Bewegungen des Auges ermitteln, um dann den für die Ablation verwendeten Laserstrahl entsprechend den Augenbewegungen zu steuern (nachzuführen). Den Stand der Technik hierzu beschreibt zum Beispiel die DE 197 02 335 C1.

Wie vorstehend erwähnt ist, sind die Verfahren der photorefraktiven Hornhautchirurgie des Standes der Technik zur Korrektur von Fehlsichtigkeit niederer Ordnung im wesentlichen "Pauschalverfahren" in dem Sinne, daß die Korrektur auf den (pauschalen) Dioptrie-Wert des Auges abstellt. Die Korrektur derartiger Fehlsichtigkeit niederer Ordnung kann zum Beispiel mit sphärischen oder astigmatischen Linsen oder auch eben mit einer photorefraktiven Korrektur der Hornhaut erfolgen.

Allerdings wird die optische Abbildung im Auge nicht nur durch die genannten Fehlsichtigkeiten niederer Ordnung beeinträchtigt, sondern auch durch sogenannte Bildfehler höherer Ordnung. Solche Bildfehler höherer Ordnung treten insbesondere auf nach operativen Eingriffen an der Hornhaut und innerhalb des Auges (Katarakt-Operationen). Solche optischen Aberrationen können die Ursache dafür sein, daß trotz einer ärztlichen Korrektur eines Fehlers niederer Ordnung die volle Sehschärfe (Visus) nicht erreicht wird. P. Mierdel, H.-E. Krinke, W. Wigand, M. Kaemmerer und T. Seiler beschreiben in DER OPHTALMOLOGE, Nr. 6, 1997, S.441 eine Messanordung zur Bestimmung der Aberration des menschlichen Auges. Mit einer solchen Messanordung können Aberrationen (Abbildungsfehler) für monochromatisches Licht gemessen werden, und zwar nicht nur durch die Hornhaut bedingte Aberrationen, sondern es können die vom gesamten okularen Abbildungssystem des Auges verursachten Abbildungsfehler gemessen werden, und zwar ortsabhängig, d.h. mit einer bestimmten Auflösung kann für gegebene Orte innerhalb der Pupille des Auges bestimmt werden, wie groß an dieser Stelle der Abbildungsfehler des gesamten optischen Systems des zu korrigierenden Auges ist. Derartige Abbildungsfehler des Auges werden in der vorstehend zitierten Arbeit von P. Mierdel et al. als sogenannte Wellenfrontaberration mathematisch beschrieben. Man versteht unter einer Wellenfrontaberration den räumlichen Verlauf des Abstands zwischen der realen Lichtwellenfront eines zentralen Lichtpunktes und einer Referenzfläche, wie z. B. ihrer idealen, kugelförmigen Gestalt. Als räumliches Bezugssystem dient also z. B. die Kugeloberfläche der idealen Wellenfront. Als Bezugssystem für die Aberrationsmessung wird eine Ebene gewählt, wenn die zu vermessende ideale Wellenfront eben ist.

Das Messprinzip gemäß der genannten Arbeit von P. Mierdel, T. Seiler et al. wird auch bei der PCT/EP00/00827 eingesetzt. Es beinhaltet im wesentlichen, daß ein Parallelstrahlbündel hinreichenden Durchmessers durch eine Lochmaske in getrennte parallele Einzelstrahlen aufgeteilt wird. Diese Einzelstrahlen durchlaufen eine Sammellinse (sogenannte Aberroskoplinse) und werden dadurch beim emmetropen Auge in einem bestimmten Abstand vor der Retina fokussiert. Die Folge sind gut sichtbare Projektionen der Maskenlöcher auf der Retina. Dieses retinale Lichtpunktmuster wird nach dem Prinzip der indirekten Ophtalmoskopie auf die Sensorfläche einer CCD-Videokamera abgebildet. Im aberrationsfreien idealen Auge ist das abgebildete Lichtpunktmuster unverzerrt und entspricht genau dem Lochmaskenmuster. Ist aber eine Aberration gegeben, kommt es zu individuellen Verschiebungen jedes Musterpunktes, weil jeder Einzelstrahl einen bestimmten Hornhaut- bzw. Pupillenbereich durchläuft und gemäß der irregulären optischen Wirkung eine Abweichung vom idealen Verlauf erfährt. Aus den retinalen Musterpunktverschiebungen wird schließlich die Wellenfrontaberration mit einem Näherungsverfahren als Ortsfunktion über der Pupillenfläche ermittelt. Der genannte Stand der Technik beschreibt auch die mathematische Darstellung dieser Wellenfrontaberration in Form eines sogenannten "Wellenfrontaberrationsgebirges". Dieses "Wellenfrontabenationsgebirge" gibt über jedem Pupillenort (x-y Koordinaten) einen Wert für die Wellenfrontaberration $W(x,y)$ an, der dann als Höhe über den x-y Koordinaten aufgetragen ist. Je höher das "Gebirge" ist, um so größer sind die Abbildungsverzehrungen im Auge an dem jeweiligen Pupillenort. Für jeden einfallenden Lichtstrahl besteht in erster Näherung eine Proportionalität zwischen der gemessenen Abweichung des entsprechenden retinalen Lichtpunktes von seiner idealen Position und der Steilheit des "Wellenfrontaberrationsgebirges". Somit kann daraus die Wellenfrontaberration als Ortsfunktion, bezogen auf einen willkürlichen Referenzwert auf der optischen Achse des Systems, bestimmt werden. Ideale, im Regelfall unverzerrte Lichtpunktpositionen auf der Retina, die den Referenzwert liefern können, sind zum Beispiel vier zentrale Punkte mit geringem gegenseitigen Abstand. Solche Punkte repräsentieren eine zentrale Hornhaut-Pupillen-Zone von etwa 1 bis 2 mm Durchmesser, die erfahrungsgemäß als weitgehend frei von Bildfehlern höherer Ordnung angenommen werden kann.

Das "Wellenfrontaberrationsgebirge" kann in verschiedener Weise mathematisch mit Hilfe eines geschlossenen Ausdruckes (einer Funktion) dargestellt werden. In Betracht kommen z. B. Approximationen in Form einer Summe von Taylor- oder auch insbesondere Zernike-Polynomen. Die Zernike-Polynome haben den Vorteil, daß ihre Koeffizienten einen direkten Bezug zu den allgemein bekannten Bildfehlern (Öffnungsfehler, Koma, Astigmatismus, Verzeichnung) haben. Die Zernike-Polynome sind ein Satz vollständig orthogonaler Funktionen. In einem Aufsatz von J. Liang, B. Grimm, S. Goelz und J. F. Bille, "Objective Measurement of Wave Aberrations of the Human Eye with the use of a Hartmann-Shack Wave-Front Sensor", Optical Society of America, 11(7):1949-1957, Juli 1994, wird gezeigt, wie die Wellenfront (bzw. Wellenfrontaberration) aus den Gitterpunktverschiebungen berechnet werden kann. Aus der Bestimmung der Ableitungsfunktion der Wellenfront läßt sich so die eigentliche Wellenfront ermitteln. Die Wellenfront ergibt sich als Lösung eines Gleichungssystems. Auch der Aufsatz von H. C. Howland und B. Howland, "A Subjective Method for the Measurement of Monochromatic Aberrations of the Eye", Journal of the Optical Society of America, 67(11): 1508-1518, November 1977, beschreibt ein Verfahren zum Bestimmen der monochromatischen Aberration und die Ermittlung der ersten fünfzehn Taylor-Koeffizienten.

Die in der oben genannten PCT/EP00/00827 vorgestellte Vorrichtung für die photorefraktive Hornhautchirurgie bei Sehfehlern höherer Ordnung weist die folgenden Einrichtungen auf:

- ein Aberroskop zum Messen der Wellenfrontaberration des gesamten optischen Systems des zu korrigierenden Auges in bezug auf eine bestimmte Augenposition,

- Mittel zum Ableiten eines Photoablationsprofils aus der gemessenen Wellenfrontaberration derart, daß eine Photoablation gemäß dem Photoablationsprofil die Wellenfrontaberration des behandelten Auges minimiert, und
- eine Laserstrahlungsquelle und Mittel zum Steuern der Laserstrahlung in Bezug auf die bestimmte Augenposition zur Abtragung des Photoablationsprofils.

Auch, wenn diese Vorrichtung gegenüber den Vorgängerlösungen bedeutende Verbesserungen hervorbrachte, erwies sich, daß die Behandlungserfolge in einigen Fällen nicht so gut waren, wie es bei der Genauigkeit, mit der das Photoablationsprofil erstellt wurde, zu erwarten gewesen wäre.

Es ist demgegenüber Aufgabe der vorliegenden Erfindung, einen Weg aufzuzeigen, wie noch bessere Behandlungserfolge erzielt werden können.

Der Erfindung liegt die Erkenntnis zugrunde, daß im Stand der Technik zwar ein sehr genaues Ablationsprofil ermittelt wurde, daß aber bei der Durchführung der Ablation vereinfachend davon ausgegangen wurde, daß der Laserstrahl an jeder Stelle der Hornhaut eine gleichmäßige Abtragung bewirkt. Der Laserstrahl trifft aber unter unterschiedlichen Winkeln auf die verschiedenen Stellen auf der Hornhaut auf. Dies hat zwei Folgen: zum einen verändert sich die Dichte der auf der Hornhautoberfläche auftreffenden Laserstrahlenergie mit diesem Winkel, zum anderen wird von der auftreffenden Laserstrahlung je nach Winkel ein unterschiedlich großer Anteil reflektiert.

Die erfindungsgemäße Vorrichtung zur Lösung der vorstehend genannten Aufgabe ist im Patentanspruch 1 beschrieben.

Nachfolgend wird ein Ausführungsbeispiel der Erfindung anhand der Zeichnungen näher erläutert. Es zeigt:

Figur 1      schematisch die Wellenfrontaberration

Figur 2      schematisch ein Aberroskop zum Messen der Wellenfrontaberration des gesamten optischen Systems eines zu behandelnden Auges;

Figur 3      schematisch eine Meß- und Steueranordnung zum Durchführen einer photorefraktiven Keratektomie des Auges mit Mitteln zum Ableiten eines Photoablationsprofils und Mitteln zum Steuern der Laserstrahlung;

Figur 4      die Abhängigkeit der Ablationstiefe von der Strahlenergiedichte;

Figur 5      schematisch die Oberfläche der Hornhaut mit auf der Oberfläche auftreffendem Laserstrahlspot und mit eingezeichneten Achsen;

Figur 6      die Abhängigkeit eines ersten Korrekturfaktors vom Abstand r des Auftreffpunkts des Laserstrahlspotmittelpunkts auf der Hornhaut zur z-Achse für verschiedene Radien R der Hornhaut;

Figur 7      schematisch die Oberfläche der Hornhaut und den im Winkel $\alpha_1$ einfallenden Laserstrahl;

Figur 8      die Abhängigkeit eines zweiten Korrekturfaktors vom Abstand r des Auftreffpunkts des Laserstrahlspotmittelpunkts auf der Hornhaut zur z-Achse verschiedene Radien R der Hornhaut;

Figur 9      die Abhängigkeit eines kombinierten Korrekturfaktors für die Ablationstiefe vom Abstand r des Auftreffpunkts des Laserstrahlspotmittelpunkts auf der Hornhaut zur z-Achse für verschiedene Radien R der Hornhaut

Figur 10     die Abhängigkeit des Verhältnisses von dem Abstand, bei dem die Dichte der auf der Hornhautoberfläche auftreffenden, nicht reflektierten Energie 80 % beträgt, zu dem Abstand, bei dem sie 0 ist, von der Strahlenergiedichte des einfallenden Laserstrahls;

Figur 11     schematisch den Strahlverlauf bei nicht zentriertem Auftreffen des Laserstrahlspots;

Figur 12     die Abhängigkeit des kombinierten Korrekturfaktors für die Ablationstiefe von dem Abstand r des Auftreffpunkts des Laserstrahlspotmittelpunkts auf der Hornhaut von der in Figur 11 gezeigten z-Achse bei unterschiedlichem Ausmaß der Dezentrierung $r_v$ bei einem Krümmungsradius von R = 7,8 mm und einer Strahlenergiedichte des einfallenden Laserstrahls von F = 150 mJ/cm$^2$.

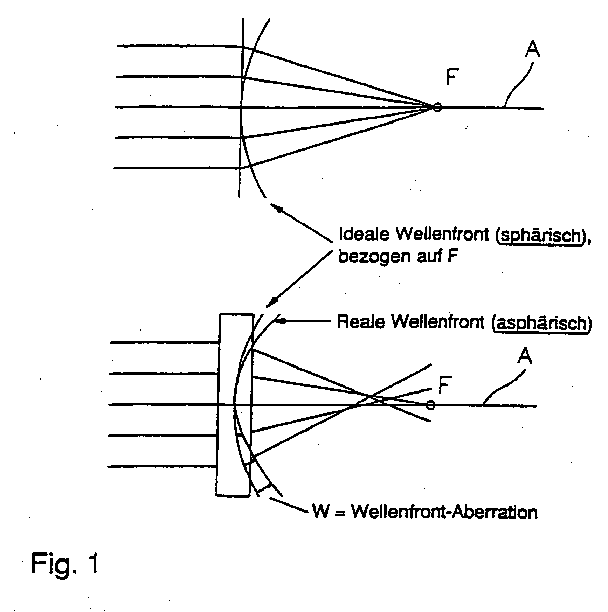

Figur 1 zeigt schematisch die oben bereits erläuterte Wellenfrontaberration eines Auges, d. h. die Abweichung der realen, asphärischen Wellenfront von der idealen Wellenfront. A ist die optische Achse des Systems und F der Brennpunkt, letzteres hier auch der gedachte Ausgangspunkt der Strahlung im Falle einer idealen Wellenfront.

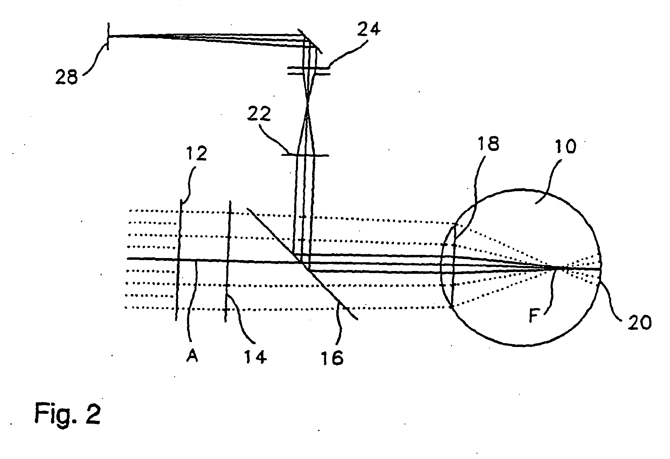

Figur 2 zeigt schematisch das optische Schema eines Video-Aberroskops zur Messung der Wellenfrontaberration eines Auges 10. Das grüne Licht eines HeNe-Lasers (543 nm) wird auf einen Durchmesser von etwa 12 mm aufgeweitet und anschließend mittels einer Lochmaske 12, in der eine Vielzahl äquidistanter Löcher ausgebildet sind, in eine entsprechende Anzahl paralleler Einzelstrahlen aufgeteilt. Gemäß Figur 2 verlaufen diese Einzelstrahlen, die nur schematisch durch punktierte Linien angedeutet sind, parallel zur optischen Achse A des Systems. Durch eine Aberroskoplinse 14 (Sammellinse) vor dem Auge 10 werden diese Strahlen so gebrochen, daß sie in einem bestimmten Abstand vor der Netzhaut 20 fokussiert werden (Fokus F). Bei einem rechtsichtigen Auge hat die Aberroskoplinse z. B. einen Brechwert von +4dpt. Im aberrationsfreien Idealauge entsteht auf diese Weise ein völlig unverzerrtes Lichtpunktmuster auf der Netzhaut 20. Die Pupille ist mit dem Bezugszeichen 18 angedeutet.

Weist das Auge 10 jedoch eine Aberration auf, so werden die Musterpunkte entsprechend den Abbildungsfehlern verschoben, da jeder Einzelstrahl nur einen ganz bestimmten Ort der Pupille 18 passiert und gemäß den irregulären optischen Wirkungen eine Abweichung vom idealen Verlauf erfährt. Diese Abweichung vom idealen Verlauf entspricht dem optischen Abbildungsfehler des gesamten optischen Systems des Auges 10 bezüglich eines Lichtstrahls, der den bestimmten Ort innerhalb der Pupille passiert. Auf der Hornhaut haben die Einzelstrahlen z. B. in x- und y-Richtung einen konstanten Abstand von 1,0 mm und ihr Durchmesser beträgt beispielhaft etwa 0,5 mm. Das gesamte parallele Meßstrahlbündel hat auf der Hornhaut z. B. eine Abmessung von 8 x 8 mm.

Mittels eines Halbspiegels 16 wird das auf der Netzhaut 20 erzeugte Lichtpunktmuster über eine Ophthalmoskoplinse 22 und ein Objektiv 24 für das Netzhautbild auf eine Sensorfläche 28 einer Festkörper-Bildkamera (CCD-Kamera) abgebildet, um das entstehende Lichtpunktmuster rechnerisch zu verarbeiten. Die Abweichungen der Orte der Lichtpunkte, bezogen auf die äquidistante, regelmäßige Struktur des fehlerfreien Auges, ergibt die Möglichkeit, die Wellenfrontaberration W (x, y) als Ortsfunktion über die Pupillenfläche des Auges zu ermitteln. Die Ortsfunktion kann mittels eines Satzes von Polynomen approximiert werden, z. B. Taylor-Polynomen oder Zernike-Polynomen. Die Zernike-Polynome werden hier bevorzugt, weil ihre Koeffizienten $C_i$ den Vorteil eines direkten Bezuges zu den Bildfehlern haben, wie Öffnungsfehler, Koma, Astigmatismus, Verzeichnung. Mit den Zernike-Polynomen $Z_i$ (x, y) läßt sich die Wellenfrontaberration W wie folgt darstellen:

$$W(x,y) = \Sigma_i \; C_i \; x \; Z_i(x,y).$$

Mit (x,y) sind die kartesischen Koordinaten in der Pupillenebene bezeichnet.

Mit der Bestimmung von z. B. den ersten 14 Koeffizienten $C_i$ (i = 1,2, ..., 14)der Zernike-Polynome ist eine hinreichend genaue Beschreibung der Wellenfrontaberration W(x,y) als Funktion der Ortskoordinaten der freien Pupillenfläche möglich. Auf diese Weise ergibt sich ein sog. Wellenfrontaberrationsgebirge, d. h. in einer dreidimensionalen Darstellung eine Funktion über den Ortskoordinaten x,y, die den jeweils lokalen Abbildungsfehler angibt. Außer den Zernike-Polynomen können auch andere Möglichkeiten gewählt werden, die Wellenfront mathematisch zu beschreiben, z. B. Taylor-Reihen. Die Zernike-Polynome sind nur das hier gewählte Ausführungsbeispiel.

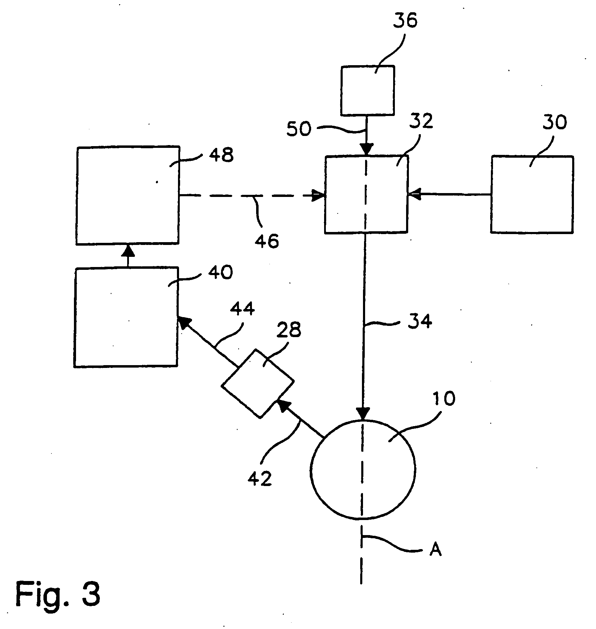

Aus dieser Wellenfrontaberration W(x,y) wird mittels eines Rechners 48 (Figur 3) ein sog. Photoablationsprofil berechnet. Der Rechner ermittelt also letztlich aus dem Lichtpunktmuster die Wellenfrontaberration in Form einer bestimmten Anzahl von Zernike-Koeffizienten und dann aus der Wellenfrontaberration ein Photoablationsprofil, d. h. Daten darüber, bis zu welcher Tiefe am jeweiligen Pupillenort die Hornhaut abgetragen (ablatiert) werden muß, um die Wellenfrontaberration zu verkleinern. Das Ablationsprofil, also die Schichtstärke des abzutragenden Materials in Abhängigkeit vom Ort (X-Y-Koordinaten) kann auf verschiedene Weise aus der Wellenfront (Aberration) bestimmt werden:

Grundsätzlich erfolgt die Berechnung des Ablationsprofils für ein zu korrigierendes Auge mit einem entsprechenden Augenmodell.

Dazu wird die Wellenfrontaberration auf die Hornhautoberfläche unter Berücksichtigung der geometrischen Eigenschaften des Auges, wie z. B. der Hornhautdicke, Abstand zwischen Hornhautrückfläche und Linsenvorderfläche, Abstand zwischen Linsenvorderfläche und Linsenrückfläche, Abstand zwischen Linsenrückfläche und Netzhaut, mathematisch projiziert. Weiterhin werden bei der Berechnung des Ablationsprofils die Brechungsindizes der einzelnen optischen Elemente des Auges berücksichtigt. Die Wellenfront beschreibt im wesentlichen die Laufzeitunterschiede des Lichts, d. h. die optische Wegstrecke. Dividiert man die optische Wegstrecke durch den Brechungsindex, so erhält man den geometrischen Weg. Es läßt sich somit aus der Projektion der Wellenfront auf die Hornhaut das zugehörige Ablationsprofil ableiten. In der Art einer Iteration wird an der gegebenen Stelle der Hornhaut eine Ablationstiefe (bei LASIK entsprechend eine Tiefe des im Stroma ablatierten Materials) mathematisch angenommen und berechnet, wie sich eine solche Ablation auf die Laufzeitunterschiede der Strahlen auswirken würde. Ziel ist eine Angleichung der Laufzeiten der Strahlen an allen Orten der Hornhaut derart, daß die Wellenfrontaberration möglichst gering wird. Dabei muß berücksichtigt werden, daß die Wellenfront auch Werte annehmen kann, die in ihrer physikalischen Bedeutung einen Auftrag von Gewebe bedeuten (d. h. eine Verstärkung der Hornhaut), was in der Regel nicht möglich ist. Deshalb muß das Ablationsprofil entsprechend angepaßt werden, d. h. insgesamt so verschoben werden, daß nur durch Ablation (Abtrag) von Gewebe das gewünschte Zielprofil der Hornhaut erreicht wird.

Die Wellenfrontaberration läßt sich nicht nur in der Pupillenebene (Eintrittspupille; englisch: entrance pupil) berechnen, sondern auch direkt an der Hornhaut. Unter Berücksichtigung der entsprechenden Brechungsindizes ergibt sich somit das eigentliche Ablationsprofil für einen bestimmten Pupillendurchmesser.

Eine Korrektur der für die Ermittlung des Ablationsprofils verwendeten Wellenfrontaberration $W(x,y)$ wird dahingehend vorgenommen, daß der Heilungsprozeß des Auges nach der Operation mitberücksichtigt wird. Der Heilungsprozeß hat nämlich eine Änderung der optischen Eigenschaften des Auges zur Folge hat und daß zur Erzielung bester Ergebnisse diese Änderungen bei der zugrundegelegten Wellenfrontaberration berücksichtigt werden sollten. Dies geschieht wie folgt:

In die obige Gleichung, in der die Wellenfrontaberration $W(x,y)$ als Summe von Zernike-Polynomen $Z_i(x,y)$ dargestellt ist, werden sog. Korrekturfaktoren ("fudge factors") $A_i$ eingeführt:

$$W(x,y) = \sum_{i=0}^{n} A_i \times C_i \times Z_i(x,y)$$

Im Vergleich zur obigen Formel sind in der Summe von Zernike-Koeffizienten und Zernike-Polynomen jeweils Korrekturfaktoren $A_i$ hinzugefügt worden, die empirisch dem Wundheilungsprozeß Rechnung tragen. Mit anderen Worten: Die vorstehende Funktion $W(x,y)$ beschreibt die zu korrigierende Wellenfront am Auge unter Berücksichtigung von postoperativen Änderungen einzelner optischer Bildfehler ($Z_i$) durch die Wundheilung. Dabei sind insbesondere klinisch relevant die Zernike-Koeffizienten von nullter bis achter Ordnung. Die Polynom-Koeffizienten $C_i$ beschreiben, wie oben bereits erläutert ist, die Größe des Bildfehlers aus der beschriebenen Messung.

Es hat sich empirisch gezeigt, daß der klinisch relevante Wertebereich der Korrekturfaktoren $A_i$ im Bereich von -1000 bis 0 bis +1000 liegt. Es wurde weiter empirisch ermittelt, daß die klinischen Korrekturfaktoren $A_i$ für jeden Koeffizienten $C_i$ unterschiedliche Werte annehmen. $A_i$ ist also eine Funktion von $C_i$. Diese funktionale Abhängigkeit $A_i = f_i(C_i)$ ist unterschiedlich für die einzelnen Koeffizienten $C_i$, d. h. die Funktion $f_i$ hat verschiedene Verläufe für die einzelnen Koeffizienten $C_i$.

Es hat sich weiter gezeigt, daß die Funktion $A_i = f_i(C_i)$ weiterhin vom jeweils verwendeten therapeutischen Lasersystem abhängig ist, da der postoperative Heilungsverlauf auch vom jeweils verwendeten Lasersystem selbst abhängig ist. Dies bedeutet, es können in der Regel keine allgemein gültigen (abstrakten) Daten oder Algorithmen für die klinischen Korrekturfaktoren $A_i$ angegeben werden, vielmehr müssen diese Korrekturfaktoren empirisch (experimentell) klinisch für das jeweils verwendete Lasersystem ermittelt werden, wobei der oben angegebene typische Wertebereich von -1000 über 0 bis +1000 gilt, insbesondere für das hier verwendete Lasersystem der Firma WaveLight, Erlangen, Deutschland.

Wie gesagt, können aufgrund der Wellenfrontaberration ermittelte Ablationsprofile, wenn die vorstehend genannten Korrekturfakten $A_i$ nicht verwendet werden, zu einer Überbewertung oder Unterbewertung einzelner Bildfehler aufgrund der Wundheilung nach dem refraktiven Eingriff führen, bei LASIK also u. a. das Anheilen des zurückgeklappten Scheibchens ("flap"). Z. B. muß für die Korrektur eines Komas von etwa $Z_7 = 0,3\ \mu m$ ein Koma von $Z_7 = 0,5\ \mu m$ von der Hornhaut abgetragen werden, damit nach dem Abschluß der Wundheilung (z. B. Epithelschluß, ca. 7 Tage) ein $Z_7 = 0$ resultiert ("Z" steht hier für den Zernike-Koeffizienten als Beispiel).

Die gemäß obiger Vorgabe ermittelten Korrekturfaktoren $A_i$ werden im Rechner abgelegt und das Computerprogramm arbeitete sie (automatisch) in das letztlich zur Anwendung kommende Ablationsprofil ein.

Alternativ zur vorstehend beschriebenen Berechnung des Ablationsprofils aus der Wellenfrontaberration kann das Ablationsprofil auch direkt aus einer Projektion von Punkten auf die Hornhaut und die Netzhaut berechnet werden. Fällt ein Lichtstrahl mit bekannten Einfallswinkeln und Koordinatenpunkten auf die Hornhaut und dann in das Auge, so wird dieser Lichtstrahl entsprechend den optischen Eigenschaften des Auges auf der Netzhaut abgebildet. Da die Position des Lichtstrahls auf der Hornhaut und die Einfallswinkel des Strahls bekannt sind, läßt sich durch Messung der Position des Lichtstrahls auf der Netzhaut der optische Strahlengang reproduzieren. Wird dabei festgestellt, daß die Position des Lichtstrahls auf der Netzhaut von der Sollposition abweicht (die Sollposition bedeutet eine aberrationsfreie Abbildung), so läßt sich aus der Positionsabweichung die Aberration ermitteln. Das Licht wird entsprechend der geometrischen Krümmung der Oberfläche der Hornhaut und den weiteren Aberrationsfehlern des Systems "Auge" gebrochen. Die vorstehend genannte Positionsabweichung des Lichtstrahls auf der Netzhaut kann durch eine entsprechende Änderung des Lichteinfallswinkels ausgedrückt werden. Der Lichteinfallswinkel ist proportional zur Ableitungsfunktion der Oberfläche der Hornhaut. Durch iteratives Vorgehen kann aus der Positionsverschiebung des Lichtstrahls auf der Netzhaut und der damit verbundenen Änderung des Lichteinfallswinkels auf eine (krankhafte) Änderung der Krümmung der Hornhautoberfläche geschlossen werden. Die Änderung der Krümmung der Hornhautoberfläche beschreibt also die Ableitungsfunktion des (gesuchten) Ablationsprofils. Wird dieses Verfahren mit einer ausreichenden Anzahl von Lichtstrahlen an unterschiedlichen Punkten des Auges durchgeführt (z. B. durch Projektion eines Gitters auf die Hornhaut), läßt sich die gesamte Ableitungsfunktion des (gesuchten) Ablationsprofils bestimmen. Hieraus kann dann mit bekannten mathematischen Verfahren (z. B. Spline-Interpolation und anschließende Integration) das Ablationsprofil berechnet werden.

Es hat sich gezeigt, daß Ablationsprofile, die mit Wellenfrontmessungen gewonnen worden sind, in einigen Fällen eine sog. Übergangszone erforderlich machen, weil ohne eine solche Übergangszone unter Umständen am Rand des Ablationsprofils ein bestimmter Rest an Material stehen bliebe, d. h. es würde sich auf der Hornhaut eine Stufe ergeben. Um eine derartige Stufe zu vermeiden, wird eine ca. 0,5 mm bis 3 mm breite Übergangszone um das Ablationsprofil herum nach außen hin vorgesehen, um eine glatte, stufenlose Fläche auf der gesamten Hornhaut zu gewährleisten.

Figur 3 zeigt schematisch das Rechner- und Steuersystem zur Durchführung einer Photoablation gemäß dem errechneten Photoablationsprofil. Die Photoablation erfolgt sowohl oberflächlich auf der Hornhaut als auch intra-stromal.

Als Laser 30 für die Photoablation kommt insbesondere in Betracht ein Excimerlaser (193 nm). Ebenfalls in Betracht kommen insbesondere Er:YAG-Festkörperlaser mit einer Wellenlänge von 2,94 $\mu$m und UV-Festkörperlaser (z.B. Nd:YAG mit 213 nm).

Die Laserstrahlung wird mittels eines galvanometrischen Abtasters (Scanner) 32 umgelenkt, und der umgelenkte Laserstrahl 34 wird auf das Auge 10 gerichtet.

Koaxial mit dem Laserstrahl 34 wird ein weiterer Strahl einer sog. Positionierlichtquelle 36 auf das Auge 10 gerichtet. Der Strahl 50 der Positionierlichtquelle 36 definiert eine Bezugsachse A, die im Raum ortsfest ist.

Im Realfall bewegt sich das Auge 10 in Bezug auf die Achse A. Um bei derartigen Bewegungen den Bearbeitungsstrahl 34 und entsprechend das abzuarbeitende Ablationsprofil den Bewegungen des Auges anzupassen (nachzuführen), wird das Auge mit Infrarotstrahlung (nicht gezeigt) beleuchtet, und mittels der CCD-Kamera 28 werden Bilder aufgenommen mit einer bestimmten Bildfolgefrequenz. Die Bildstrahlung 42 des Auges erzeugt also in der CCD-Kamera 28 Bilder, die elektronisch verarbeitet werden. Das elektronische Ausgangssignal 44 der Kamera 28 wird einer Bildverarbeitungseinrichtung 40 zugeführt, und das Ergebnis der Bildverarbeitung wird in einen Rechner 48 eingegeben, der sowohl die Auswertung als auch die Steuerung des Scanners 32 übernimmt. Der Rechner 48 gibt also ein entsprechendes Stellsignal 46 an den Scanner (Abtaster) 32, so daß der Laserstrahl 34 so gesteuert wird, daß in Bezug auf eine bestimmte Augenposition, in Bezug auf die auch die Wellenfrontablation gemessen worden ist, auch das Ablationsprofil abgearbeitet wird. Auf diese Weise können die optischen Fehler des gesamten Auges durch Photoablation der Hornhaut korrigiert werden. Das hier im vorstehenden Sinne abgearbeitete Ablationsprofil ist das aus der Wellenfrontmessung gewonnene und um die oben erläuterten empirischen Korrekturfaktoren aufgrund der Wundheilung abgeänderte Ablationsprofil.

Die bisher beschriebene Vorrichtung ist auch der PCT/EP00/00827 zu entnehmen. Damit das so aufwendig errechnete Photoablationsprofil auch umgesetzt wird, wird nun der Rechner 48 gemäß der vorliegenden Erfindung so programmiert, daß der Einfluß des Winkels zwischen Laserstrahl und Hornhautoberfläche auf die Ablationstiefe berücksichtigt wird.

Wie bereits erwähnt, spielen dabei zwei Faktoren eine Rolle:

1) Der Laserstrahlspot (Laserstrahlfleck) verändert seine Größe und Form winkelabhängig beim Auftreffen auf eine gekrümmte Oberfläche, wodurch sich die Energiedichte des auftreffenden Laserstrahls verändert, und
2) je nach dem Winkel zwischen dem Laserstrahl und der Hornhautoberfläche wird ein unterschiedlicher Anteil der auftreffenden Energie des Lasers wegreflektiert.

Somit verringert sich die wirksame, d.h. die ablatierende Energiedichte in Abhängigkeit vom Winkel zwischen Laserstrahl und Hornhautoberfläche.

Abhängigkeit der Ablationstiefe von der wirksamen Energiedichte

Zunächst muß daher untersucht werden, wie sich die unterschiedliche wirksame Energiedichte auf die Ablationstiefe auswirkt.

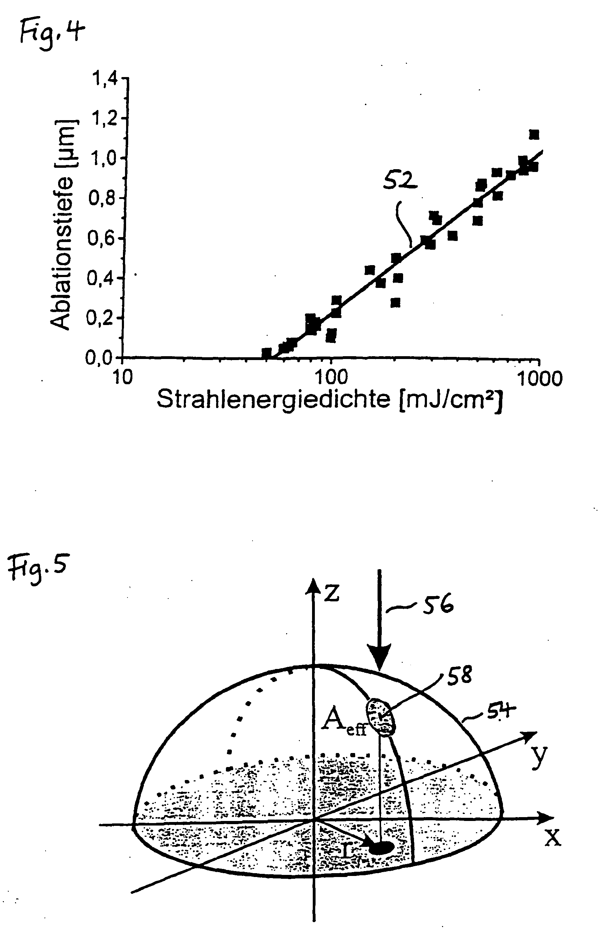

Dies ist in Figur 4 dargestellt. Die quadratischen Punkte stehen dabei für gemessene Werte bei Laserpulsen einer bestimmten Dauer (für eine Laserstrahlung eines ArF-Excimerlasers mit 193 nm Wellenlänge). Man kann erkennen, daß die Ablationstiefe mit dem Logarithmus der wirksamen Strahlenergiedichte ansteigt. Die Ablationstiefe d folgt somit der Formel

$$d = m \cdot \ln\left(\frac{F}{F_o}\right), \qquad\qquad (1)$$

wobei F die wirksame Strahlenergiedichte und $F_{th}$ ein Energiedichtenschwellwert ist, ab dem überhaupt eine Ablation erst einsetzt. Der Faktor m ist eine Konstante. Entsprechend dieser Formel wurde die Kurve 52 gefittet. Der Energiedichtenschwellwert $F_{th}$ ergab sich dabei zu 50 mJ/cm$^2$.

Die Tatsache, daß die Ablationstiefe in Abhängigkeit von der wirksamen Strahlenergiedichte einer solch einfachen Formel folgt, erleichtert eine numerische Verarbeitung in dem Rechner 48.

Auftreffende Energiedichte bei Auftreffen auf die gekrümmte Oberfläche

Im folgenden soll nun untersucht werden, wie sich die auftreffende Energiedichte in Abhängigkeit vom Ort des Auftreffens des Laserstrahlspots auf der Hornhaut ändert.

In Figur 5 ist schematisch die als sphärisch angenommene Hornhaut 54 gezeigt, auf die ein Laserstrahl 56 auftrifft. Hier ist zur Vereinfachung zunächst angenommen, daß der Laserstrahl 56 parallel zur z-Achse strahlt. Der Laserstrahlspot hat auf der Oberfläche der Hornhaut 54 eine Fläche $A_{eff}$.

Die Fläche $A_{eff}$ kann nun in Abhängigkeit von den Koordinaten des Auftreffpunkts des Laserstrahlspotmittelpunkts auf der Hornhaut 54 berechnet werden.

Dabei sind nur zwei der Koordinaten unabhängig voneinander, die dritte Koordinate ergibt sich aus der Form der Oberfläche der Hornhaut 54. So gilt für die z-Koordinate in Abhängigkeit von den Koordinaten x und y:

$$z = f(x, y) = f(r) = \sqrt{R^2 - x^2 - y^2} = \sqrt{R^2 - r^2} . \qquad\qquad (2)$$

Dabei ist R der Radius der Hornhauthalbsphäre. $r = \sqrt{(X^2 + Y^2)}$ ist der Abstand von der z-Achse zum Auftreffpunkt 58 des Laserstrahlspotmittelpunkts.

Ist $r_s$ der Radius des Laserstrahlspots vor dem Auftreffen auf der Hornhaut, so erhält man für $A_{eff}(r)$:

$$A_{eff}(r) = \int_{-r_s}^{r_s} \int_{\sqrt{r_s^2 - r^2} - r}^{\sqrt{r_s^2 - r^2} + r} \sqrt{1 + \left(\frac{d}{dx} f(x,y)\right)^2 + \left(\frac{d}{dy} f(x,y)\right)^2} \, dx\, dy . \qquad (3)$$

(Diese Formel ergibt sich aus Kapitel F in: Höhere Mathematik griffbereit, Akademie-Verlag, Berlin, 1972, Seiten 638 bis 643.)

Somit ist die Fläche $A_{eff}$ auf der Hornhaut 54 um einen Faktor k1(r),

$$k1(r) = \frac{A_{eff}(r)}{A_o} = \frac{A_{eff}(r)}{\pi \cdot r_s^2} , \qquad\qquad (4)$$

größer als die Fläche $A_0$ eines senkrecht einfallenden Laserstrahlspots.

Die Strahlenergiedichte ist ja nun als Quotient aus der Pulsenergie des Lasers E und der bestrahlten Fläche A definiert, F = E/A. Somit verringert sich die Dichte der auf der Hornhautoberfläche auftreffenden Energie auf den Wert F/k1(r) gegenüber der Energiedichte F des einfallenden Laserstrahlspots.

Somit läßt sich mit der bekannten logarithmischen Abhängigkeit der Ablationstiefe von der wirksamen Strahlenergiedichte ein Korrekturfaktor kor1(r) aufstellen, mit dem die bei senkrechtem Auftreffen des Laserstrahlspots erzielte Ablationstiefe multipliziert werden muß, um die Ablationstiefe zu erhalten, wie sie im Falle, wie er in Figur 5 dargestellt ist, erzielt wird. Dieser erste Korrekturfaktor ergibt sich zu:

$$kor\,l(r) = \frac{ln\left(\dfrac{F}{k\,l(r)\,F_o}\right)}{ln\left(\dfrac{F}{F_o}\right)} \quad . \tag{5}$$

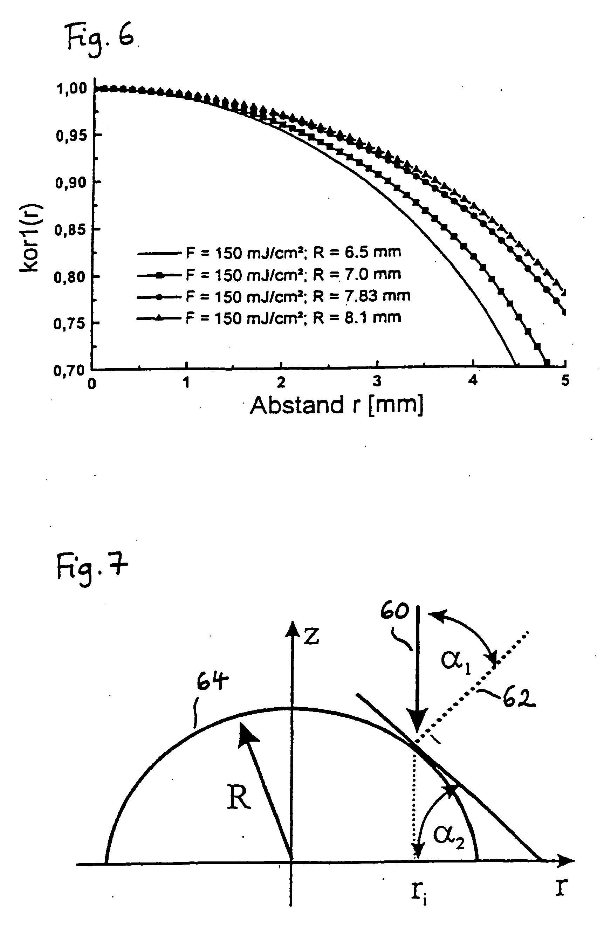

Figur 6 zeigt hierzu numerisch ermittelte Kurven für unterschiedliche Radien R der Hornhaut 54.

Wie aus Figur 6 zu erkennen, ergibt sich zum Rand der Hornhaut hin eine beträchtliche Abweichung der Ablationstiefe vom Wert 1, der bei den Verfahren des Standes der Technik vorausgesetzt wurde.

Der Rechner 48 ist so programmiert, daß er diese verringerte Ablationstiefe kompensiert, d.h., daß z. B. entsprechend mehr Laserstrahlpulse auf die betreffenden Stellen gesandt werden, damit das gewünschte Photoablationsprofil erzielt wird.

Einfluß der Oberflächenreflexion

Im folgenden betrachten wir nun die Abhängigkeit der Ablationstiefe von der Oberflächenreflexion. In Figur 7 ist der Einfallswinkel $\alpha_1$ zwischen dem auftreffenden Laserstrahl 60 und der Flächennormalen 62 zur Hornhaut definiert, wobei die Hornhaut hier schematisch im Schnitt als Halbkreis 64 dargestellt ist.

Für die Bestimmung des reflektierten Anteils des auftreffenden Lichts dienen die Fresnel-Gleichungen, die man beispielsweise dem Lehrbuch der Experimentalphysik von Bergmann, Schaefer, Band III Optik, Walter de Gruyter, Berlin, New York 1987, Seite 496 entnehmen kann:

$$q_\perp(\alpha_1) = \frac{\sqrt{n^2 - sin^2(\alpha_1)} - cos(\alpha_1)}{1 - n^2} \tag{6}$$

$$q_\parallel(\alpha_1) = \frac{n^2\,cos(\alpha_1) - \sqrt{n^2 - sin^2(\alpha_1)}}{n^2\,cos(\alpha_1) + \sqrt{n^2 - sin^2(\alpha_1)}} \quad , \tag{7}$$

wobei $q_\perp$ für senkrecht polarisiertes Licht und $q_\parallel$ für parallel polarisiertes Licht steht und n der Brechungsindex des Hornhautmaterials ist, der beispielsweise für eine Wellenlänge von 193 nm, n = 1,52 beträgt (siehe G.H. Pettit, M.N. Ediger, Cornealtissue absorption coefficients for 193- and 213-nm ultraviolet radiation, Appl. Optics 1996, Band 35, Seiten 3386 bis 3391). Um eine Abhängigkeit vom Abstand r zu erhalten, benutzt man die Formel

$$\alpha_1(r) = atan\left(\frac{r}{\sqrt{R^2 - r^2}}\right) \quad , \text{ wobei gilt: } \quad 0 \le r' < R. \tag{8}$$

Bei nicht polarisiertem Licht ergibt sich die Reflektanz k2(r) an der Grenzfläche von Luft und Gewebe zu:

$$k2(r) = \frac{q_\perp^2(r) + q_\parallel^2(r)}{2} \quad .$$

Würde man nur berücksichtigen, daß ein Teil der einfallenden Strahlung wegreflektiert wird und die oben besprochene Verringerung der Energiedichte aufgrund der Vergrößerung der effektiven Fläche $A_{eff}$ gegenüber der ursprünglichen Fläche $A_0$ beiseite lassen, so ergäbe sich eine wirksame Strahlenergiedichte von $(1 - k2(r)) \times F$ gegenüber

der einfallenden Strahlenergiedichte F und somit eine Abschwächung der Ablationstiefe d auf kor2(r) x d, wobei

$$kor2(r) = \frac{\ln\left(\frac{(1 - k2(r)) \cdot F}{F_a}\right)}{\ln\left(\frac{F}{F_a}\right)} \qquad . \qquad\qquad (9)$$

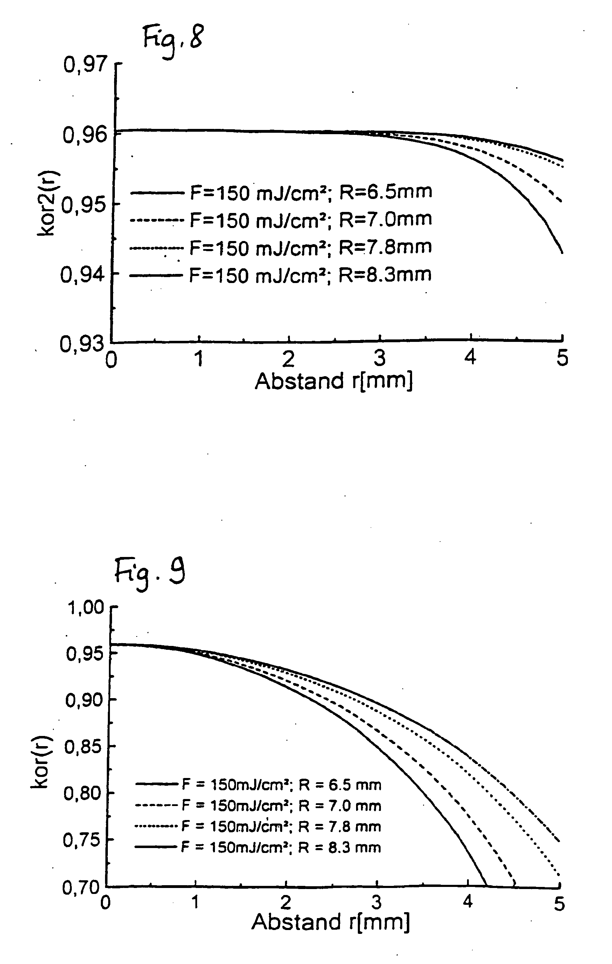

In Figur 8 sind numerisch ermittelte Kurven dargestellt, die den Verlauf von kor2 in Abhängigkeit vom Abstand r des Auftreffpunkts des Laserstrahlspotmittelpunkts auf der Hornhaut von der z-Achse für verschiedene Radien R der Hornhaut 64 dargestellt. Wie zu sehen, ist der Abfall der Ablationstiefe besonders am Rand sehr stark ausgeprägt, so daß beim Stand der Technik, bei der auch am Rand ein Wert für kor2(r) von 1 angenommen wurde, die Fehler offensichtlich besonders groß waren.

Kombination der genannten Effekte

Natürlich sollten die beiden besprochenen Phänomene der Vergrößerung der effektiven Fläche des Laserstrahlspots und der Reflektion miteinander kombiniert betrachtet werden:

Von der einfallenden Strahlenergiedichte F trifft eine Strahlenergiedichte F/k1(r) auf der Hornhautoberfläche auf, und davon wird der Anteil (1 - k2(r)) x F/k1(r) nicht wegreflektiert.

Somit ergibt sich ein kombinierter Korrekturfaktor kor(r) für die Ablationstiefe zu:

$$kor(r) = \frac{\ln\left(\frac{(1 - k2(r)) \cdot F}{k1(r) \cdot F_a}\right)}{\ln\left(\frac{F}{F_a}\right)} \qquad . \qquad\qquad (10)$$

Der Verlauf dieses kombinierten Korrekturfaktors kor(r) ist in Figur 9 anhand numerisch ermittelter Werte in Abhängigkeit von dem Abstand r des Auftreffpunkts des Laserstrahlspotmittelpunkts auf der Hornhaut von der z-Achse für unterschiedliche Krümmungsradien der Hornhaut bei einer Strahlenergiedichte von 150mJ/cm$^2$ dargestellt.

Wie in Figur 9 zu erkennen, wird der Abfall des Korrekturfaktors kor(r) zum Rand hin desto steiler, je geringer der Krümmungsradius der Hornhautoberfläche ist.

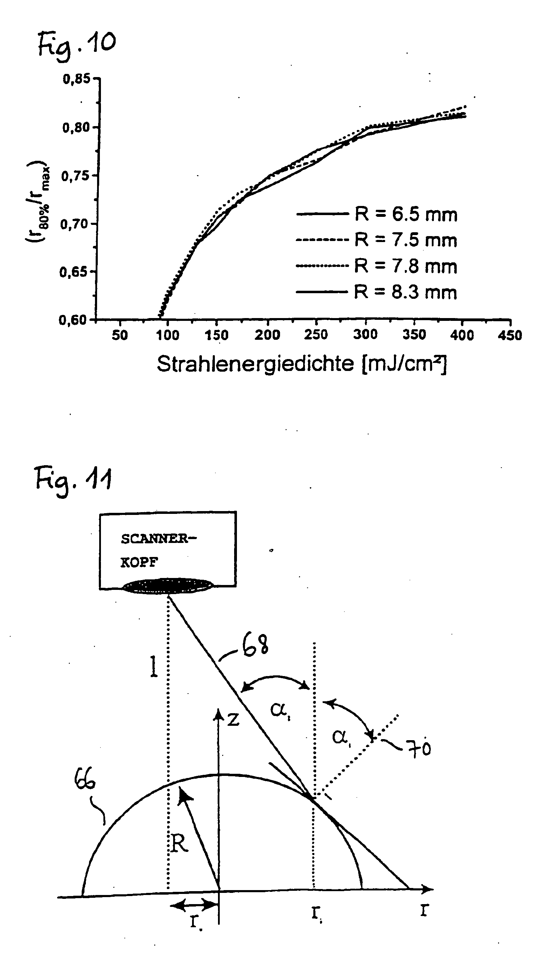

Dies kann man auch anhand von Figur 10 sehen. Dort ist der Abstand $r_{80\%}$, bei dem kor($r_{80\%}$,) = 0,8 ins Verhältnis zum Wert $r_{max}$ gesetzt, bei dem kor($r_{max}$) = 0 ist, und dieses Verhältnis ist gegen die Energiedichte des einfallenden Laserstrahls aufgetragen.

Mit der Näherung, daß der Laserstrahlspot parallel zur z-Achse eingestrahlt wird, können bereits zufriedenstellende Ergebnisse erzielt werden. Wird der Laserstrahl mittels des Steuerprogramms im Rechner 48 so gesteuert, daß die Verringerung der Ablationstiefe am Rand der Hornhaut ausgeglichen wird, können befriedigendere Heilergebnisse bei der Patientenbehandlung erzielt werden.

Schräger Einfall des Laserstrahls

Bei einem realen System ist jedoch eher die in Figur 11 gezeigte Situation gegeben. Der Kopf des in Figur 3 dargestellten, den Laserstrahl 34 auf das Auge 10 umlenkenden galvanometrischen Abtasters (Scanners) 32 sitzt gegenüber der hier schematisch als Halbkreis gezeichneten Hornhaut 66 versetzt. Ein senkrecht von dem Scannerkopf ausgehender Laserstrahl würde in einem Versetzungsabstand $r_v$ von der z-Achse auf der Hornhaut auftreffen. Außerdem ist zu sehen, daß sich der Winkel zwischen dem auf die Hornhaut 66 gesandten Laserstrahl 68 und der Flächennormalen 70 von $\alpha_1$ auf den Winkel $\alpha_1 + \alpha_2$ erhöht. Damit kommt es auf der in Figur 11 rechten Seite zu einer stärkeren Abschwächung der Ablationstiefe.

Zur Berechnung dieser Ablationstiefe muß man nur das Koordinatensystem so drehen, daß die z-Achse wieder parallel zum Laserstrahl verläuft. Dann können die oben angegebenen Formeln wieder angewendet werden.

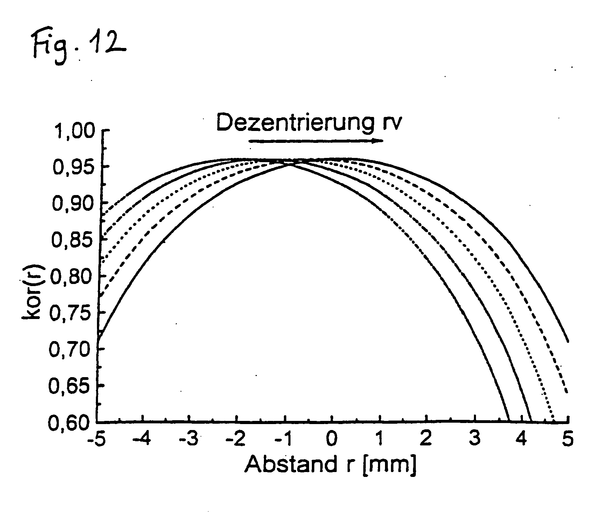

Die Abhängigkeit des Korrekturfaktors kor(r) von dem Versetzungsabstand $r_v$ ist für die in Fig. 11 gezeigte Anordnung der Hornhaut numerisch simuliert in Figur 12 dargestellt. Mit steigendem Versetzungsabstand $r_v$ nimmt der

Korrekturfaktor kor(r) einem immer asymmetrischeren Verlauf an.

Ein perfektes System berücksichtigt somit nicht nur die Vergrößerung der effektiven Fläche des Laserstrahlspots und die Reflektion an der Hornhautoberfläche, sondern auch den Einfluß der Versetzung. Dies läßt sich darin zusammenfassen, daß der Einfluß des Winkels zwischen Laserstrahl und Hornhautoberfläche berücksichtigt wird.

Nicht immer ist dieser Winkel jedoch genau bekannt. Die Erfindung kann daher noch verfeinert werden.

Erstens ist die Näherung, daß die Hornhaut sphärisch ist, im allgemeinen nicht zutreffend. Sie ist im allgemeinen asphärisch und hat auch oft einen Astigmatismus. Die Hornhaut hat somit an verschiedenen Stellen unterschiedliche Krümmungsradien. Man kann diese Krümmungsradien mit sogenannten Topographie-Systemen vermessen. Mit dieser Information über den lokalen Krümmungsradius kann nun einerseits der Winkel zwischen Laserstrahl und Hornhautoberfläche berechnet werden, wobei man Formel (8) verwendet. Damit kann man den Anteil der auf die Hornhautoberfläche auftretenden Laserstrahlenergie, der wegreflektiert wird, aus den Formeln (6) und (7) ableiten. Zur Berechnung des Einflusses des Winkels zwischen Laserstrahl und Hornhautoberfläche auf die Energiedichte des auf die Hornhautoberfläche auftreffenden Laserstrahlspots können weiterhin die Formeln (2) und (3) verwendet werden, wobei R nun der lokale Krümmungsradius der Hornhaut ist.

Zur weiteren Verfeinerung sollte berücksichtigt werden, daß sich der Winkel zwischen Laserstrahl und Hornhautoberfläche auch während des Ablatierens ändert. Wäre beispielsweise nach bisheriger Berechnung eine Folge von 50 Laserstrahlspotpulsen notwendig, um an einer bestimmten Stelle der Hornhaut gemäß dem Ablationsprofil zu ablatieren, so kann es sein, daß sich der Winkel zwischen Laserstrahl und der Hornhautoberfläche nach jedem Puls und somit jeder Teilabtragung weiter derart ändert, daß beispielsweise ein geringerer Anteil des Laserstrahls wegreflektiert wird, so daß anstelle von 50 Pulsen nur 49 oder 48 Pulsen notwendig sind, oder es kann umgekehrt sein, daß während des Ablatierens die Bedingungen ungünstiger werden, so daß anstelle der ursprünglich berechneten 50 Pulse mehr Pulse notwendig sind. Da nach den bisher angegebenen Formeln bekannt ist, um wieviel ein Laserstrahlpuls die Hornhaut ablatiert, kann dieser Effekt bei einer verfeinerten Rechnung bereits im vorhinein berücksichtigt werden. Für diese Berechnung kann die Hornhautoberfläche im Computer simuliert werden, oder es können die sich ändernden lokalen Krümmungsradien der Hornhaut näherungsweise berechnet werden. Dann würde die Berechnung genauso durchgeführt werden wie im im letzten Absatz diskutierten Falle, daß man lokal verschiedene Krümmungsradien einbezieht.

Wie oben bei der Beschreibung von Fig. 3 bereits erwähnt, werden die Bewegungen des Auges bei der Ablation verfolgt. Aufgrund dieser Bewegungen muß natürlich nicht nur das abzuarbeitende Ablationsprofil nachgeführt werden und der Scanner 32 entsprechend gesteuert werden, sondern es ändert sich dabei auch der Winkel zwischen dem Laserstrahl und Hornhautoberfläche. Bevorzugt wird auch diese Änderung berücksichtigt. Es kann der Winkel in Bezug auf die Achse A berechnet werden, und daraus kann der Winkel zwischen Laserstrahl und Hornhautoberfläche abgeleitet werden.

Die vorliegende Erfindung wurde unter Bezug auf ein System mit Fleck-Abtasten beschrieben, sie ist aber auch bei der Vollabtragung anwendbar wie auch bei der Abtragung mit Scanning-Slit (Schlitzabtragung).

Für die verwendeten Formeln können numerisch übliche Näherungen benutzt werden.

## Patentansprüche

1. Vorrichtung zum Führen eines Laserstrahls orts- und zeitgesteuert über eine zu korrigierende Hornhaut mit einer Laserstrahlquelle (30), einer Einrichtung (32) zum Richten des Laserstrahls auf das Auge (19) und mit einem Rechner (48) zum Steuern der Laserstrahlquelle (30) und der Einrichtung (32) zum Richten des Laserstrahls (34), wobei der Rechner (48) so programmiert ist, dass in einem Steuerprogramm, gemäß dem der Laserstrahl geführt wird, der Einfluss des Winkels zwischen Laserstrahl und Hornhautoberfläche auf die Energiedichte des auf die Hornhautoberfläche auftreffenden Laserstrahls und auf den von der Hornhautoberfläche reflektierten Anteil der Strahlung berücksichtigt ist, wobei berücksichtigt wird, daß sich der Winkel zwischen Laserstrahl und Hornhautoberfläche auch während des Ablatierens ändert, wobei nach bisheriger Berechnung eine Folge von Laserstrahlspotpulsen notwendig ist, um an einer bestimmten Stelle der Hornhaut gemäß dem Ablationsprofil zu ablatieren, wobei sich der Winkel zwischen Laserstrahl und der Hornhautoberfläche nach jedem Puls und somit jeder Teilabtragung weiter derart ändert, daß anstelle der ursprünglich berechneten Pulse weniger Pulse notwendig sind, oder daß anstelle der ursprünglich berechneten Pulse mehr Pulse notwendig sind, wobei bekannt ist, um wieviel ein Laserstrahlplus die Hornhaut ablatiert, so dass die bekannte Hornhautablation bei der Berechnung bereits im vorhinein berücksichtigt wird, wobei die Ablation und die Hornhautoberfläche im Computer simuliert werden.

## Claims

1. A device for guiding a laser beam, controlled with respect to position and time, over a cornea to be corrected, comprising a laser radiation source (30), means (32) for directing the laser beam onto the eye (19) and a computer (48) for controlling the laser radiation source (30) and the means (32) for directing the laser beam (34), the computer (48) being programmed such that in a control program, according to which the laser beam is guided, the effect of the angle between the laser beam and the corneal surface on the energy density of the laser beam incident on the corneal surface and on the fraction of the radiation reflected by the corneal surface is taken into account, wherein the fact that the angle between the laser beam and the corneal surface also changes during the ablation is taken into account, wherein a sequence of laser-beam spot pulses is necessary according to a previous calculation in order to ablate the cornea according to the ablation profile at a particular location, wherein the angle between the laser beam and the corneal surface changes further, after each pulse and therefore each partial ablation, so that less pulses are needed instead of the originally calculated pulses or so that more pulses are needed instead of the pulses originally calculated, wherein the extent to which a laser-beam pulse ablates the cornea is known, so that the known cornea ablation is taken into account a priori in the calculation, wherein the ablation and the corneal surface are simulated on the computer.

## Revendications

1. Dispositif pour guider dans l'espace et dans le temps un faisceau laser au-dessus de la cornée à corriger d'un oeil, comprenant une source (30) de faisceau laser, un dispositif (32) pour orienter le faisceau laser sur l'oeil (19), et un ordinateur (48) pour commander ladite source (30) du faisceau laser ainsi que ledit dispositif (32) pour orienter le faisceau laser (34), l'ordinateur (48) étant programmé de telle sorte que le programme de gestion, conformément auquel est guidé le faisceau laser, tient compte de l'influence qu'exerce l'angle entre le faisceau laser et la surface cornéenne sur la densité d'énergie du faisceau laser frappant la surface cornéenne et sur la part de rayonnement réfléchie par la surface cornéenne, dans lequel on tient compte du fait que l'angle entre le faisceau laser et la surface cornéenne varie également au cours de l'ablation, dans lequel, d'après le calcul actuel, une suite d'impulsions du spot de faisceau laser est nécessaire pour produire, en un endroit déterminé de la cornée, une ablation conformément au profil d'ablation, l'angle entre le faisceau laser et la surface cornéenne continuant à varier après chaque impulsion et donc après chaque enlèvement partiel de matière, de sorte qu'à la place des impulsions initialement calculées, moins d'impulsions sont alors nécessaires, ou qu'à la place des impulsions initialement calculées, plus d'impulsions sont alors nécessaires, dans lequel on connaît la valeur d'ablation de la cornée produite par une impulsion du faisceau laser, de sorte que la valeur connue d'ablation de la cornée est déjà préalablement prise en considération lors du calcul, l'ablation et la surface cornéenne étant simulées dans l'ordinateur.

Ideale Wellenfront (sphärisch),
bezogen auf F

Reale Wellenfront (asphärisch)

W = Wellenfront-Aberration

Fig. 1

Fig. 2

Fig. 3

Fig. 4

Fig. 5

Fig. 6

Fig. 7

Fig. 8

Fig. 9

Fig. 10

Fig. 11

Fig. 12

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19702335 C1

- EP 0000827 W, P. Mierdel, T. Seiler

**In der Beschreibung aufgeführte Nicht-Patentliteratur**

- **AUFSATZ G.F. MARSHALL.** *LASER FOCUS WORLD,* Juni 1994, 57
- **T. SEILER ; J. WOLLENSAK.** *LASERS AND LIGHT IN OPTHALMOLOGY,* 1993, vol. 5 (4), 199-203
- **J.K. SHIMMICK ; W.B. TELFAIR et al.** *JOURNAL OF REFRATIVE SURGERY,* Mai 1997, vol. 13, 235-245
- **J. LIANG ; B. GRIMM ; S. GOELZ ; J. F. BILLE.** Objective Measurement of Wave Aberrations of the Human Eye with the use of a Hartmann-Shack Wave-Front Sensor. *Optical Society of America,* Juni 1994, vol. 11 (7), 1949-1957
- **H. C. HOWLAND ; B. HOWLAND.** A Subjective Method for the Measurement of Monochromatic Aberrations of the Eye. *Journal of the Optical Society of America,* November 1977, vol. 67 (11), 1508-1518
- **KAPITEL F.** Höhere Mathematik griffbereit. Akademie-Verlag, 1972, 638-643
- **G.H. PETTIT ; M.N. EDIGER.** Cornealtissue absorption coefficients for 193- and 213-nm ultraviolet radiation. *Appl. Optics,* 1996, vol. 35, 3386-3391